Europäisches Patentamt

European Patent Office

Office européen des brevets

(11) Publication number: **0 328 588 B1**

# EUROPEAN PATENT SPECIFICATION

(45) Date of publication of patent specification: **12.01.94**  (51) Int. Cl.⁵: **G01N 33/531**, G01N 33/569, G01N 33/546

(21) Application number: **88906939.9**

(22) Date of filing: **28.07.88**

(86) International application number:
**PCT/GB88/00615**

(87) International publication number:
**WO 89/01154 (09.02.89 89/04)**

(54) **DETERMINATION OF IgM ANTIBODIES.**

(30) Priority: **28.07.87 GB 8717863**

(43) Date of publication of application:
**23.08.89 Bulletin 89/34**

(45) Publication of the grant of the patent:
**12.01.94 Bulletin 94/02**

(84) Designated Contracting States:
**BE CH DE FR GB IT LI LU NL SE**

(56) References cited:
**EP-A- 40 572**
**EP-A- 189 389**
**EP-A- 194 156**
**FR-A- 2 495 326**

**JOURNAL OF IMMUNOLOGY, vol. 131, no. 2,
August 1983; S.D.SHARMA et al., pp. 977-983***

**INFECTION AND IMMUNITY, vol. 27, no. 2,
February 1980; J.R.MINEO et al., pp. 283-287***

**INFECTION AND IMMUNITY, vol. 41, no. 1,
July 1983; Y.NAOT et al., pp. 331-333***

**CHEMICAL ABSTRACTS, vol. 99, 1983, Co-
lumbus, OH (US); no. 156726w***

**PATHOLOGY, vol. 17, 1985; A.M.JOHNSON,
pp. 9-19***

(73) Proprietor: **INTERNATIONAL INSTITUTE OF
CELLULAR & MOLECULAR PATHOLOGY
75, 50 avenue Hippocrate 75
B-1200 Brussels(BE)**

(72) Inventor: **CAMBIASO,Cesar Lorenzo, Inst.of
Cel.&Molec.Pathol.
75, 50, avenue Hippocrate 75
B-1200 Brussels(BE)**

(74) Representative: **Wain, Christopher Paul et al
A.A. THORNTON & CO.
Northumberland House
303-306 High Holborn
London WC1V 7LE (GB)**

**Description**

This invention relates to a method of assaying IgM antibodies in a sample containing a mixture of immunoglobulins. Such assays are difficult because of interference by one class of antibody in an assay for the other.

When a person becomes infected by a bacterial, viral or fungal agent the body rapidly develops an immune response, that is, the body produces macro proteins called "immuno globulins" (Ig) which attach to the infecting agent as a mechanism in its eventual destruction.

Of importance are three Ig namely IgM, IgA, IgG. At the onset of infection, the first immunoglobulins produced in vivo are of the IgM class. After reaching a peak, the concentration of IgM falls off over a relatively short time while the level of IgG rises, reaches a plateau and sometimes over a period of many years decreases slowly in level. Therefore if a high concentration of IgM is present, the infection is recent. If the concentration is exclusively IgG, the infection is old and if the concentration of IgG is high, the person has a good immunity against the infection. In many cases , the doctor needs to know if the infection is recent or old and if old, that the patient has a resistance against recurrence, the extent of that resistance being the concentration of IgG.

One very important infection of both animals and humans, toxoplasmosis, is caused by a parasite, a protozoa, Toxoplasma gondii, carried by such animals as cats. It is estimated that between 20% and 40% of the adult population in Great Britain has at one time or another been infected by this parasite and, in some parts of the world, the figure may be as high as 95%. In children and adults the symptoms of this disease resemble glandular fever and usually take a benign course. However cases of nephritis, pneumonia and rashes have been reported.

In pregnancy, the consequences of a mother having an acute (recent) infection of toxoplasmosis can be damaging to the foetus leading to possible encephalitis, hydrocephalitis, cerebral calcification, eye and ear lesions and still born babies.

More and more doctors therefore, are screening their patients for possible toxoplasmosis. Their questions are two. Has the patient:-

1. an acute toxoplasmosis that is, a high IgM or

2. an old infection and if an old infection, has the patient good protection against acquiring toxoplasmosis during pregnancy that is, high IgG?

Many methods exist today to measure the total Ig antibodies, that is, IgG and IgM. In some of these methods the infecting agent is destroyed and broken up at cellular level into fragments containing the antigen, or molecule to which IgM or IgG antibodies will bind. These fragments are then absorbed onto the wall of a plastic test tube and a sample of serum which may contain antibodies IgG or IgM is added to the tube. After a period of incubation which may be for several hours, the serum is poured out, the tube is carefully washed and then a solution containing labelled antibodies against human IgM or IgG is added.

The anti IgM or IgG antibodies have been labelled with one of the labels to which the measuring system is adapted, fluorescence or isotopes or enzymes. After another period of incubation, the contents of the tube are again emptied out and the tube is washed. Where the method involves an enzyme marked anti-Ig, a substrate solution is now added and after further period of incubation, the developed colour is measured, the optical density being proportional to the amount of IgM or IgG antibodies attached to the wall. In principle therefore one should be able to estimate the amount of either IgG or IgM present.

This method, although most commonly used today suffers from two major sources of error. IgG antibodies can exist in concentration 100 times and more greater than IgM antibodies and so high IgG antibody concentrations may block the antigens on the surface of the tube from the IgM antibodies giving therefore a falsely low value of IgM and a diagnosis that the infection is old.

An additional complication is that "C1q" (a complement protein present in all serum samples) and rheumatoid factor "RF" (which is present in samples from some patients with autoimmune diseases) bind to immune complexes formed between antibodies and antigens. After the IgG antibody has reacted with the antigen on a tube wall or a latex particle in an assay, the RF or C1q will react with the IgG. Consequently when anti IgG labelled antibody is added, much of this labelled material cannot react and the assay for IgG antibodies against the infection will be falsely low.

Several methods have been developed based on the above described assay to reduce or eliminate these interferences or causes of erroneous results (see for example Naot Y and Remington J.S.; J. Infect. Dis. 142, No. 5,757-766, 1980 or Wielaard F. et al. J. clin. Microbiology, 17, No.6, 981-87, 1983 or Cesbron Y.J. et al. J. of Immun. Methods, 83, 151-58, 1985. Since all these methods use an enzyme as a marker, they are called ELISA tests, Enzyme Linked Immunosorbent Assay. The method described in these papers has been commercialised by Organon Teknika N.V., Viedijk 58, 2300 Turnhout, Belgium under the trade name Toxonostika. The assay is however complicated, work-intensive and hence, error prone. In contrast, the method we now describe is simple, rapid, free of interference from RF or Clq, specific and is based on identifying and making the antigen from the infecting agent specific to IgM antibody.

While in their detailed structure all antigens are different, they can be said to comprise one or more of four molecular structures, glycoprotein (sugar-protein), protein, lipo-protein (fat protein) or lipopolysaccharide (fat-sugar). It has been proposed that with certain antigens from toxoplasma gondii the IgM antibodies will react with the polysaccharide and protein moieties while the the IgG antibodies would react preferentially with the protein (Mineo J.R. et al. Infect. Immun. 27, 283-287, 1980 and Camargo M.E. et al ibid 21, 55-58, 1978).

The cited authors claimed that the selective reaction of IgM and IgG antibodies for the polysaccharide and protein respectively was determined using an ELISA technique, implying therefore that the method was capable of separating the IgM from the IgG antibodies. They had separated the surface antigen toxoplasma gondii and physically denatured the protein, leaving only the polysaccharide moiety to which, they claimed, only IgM was attached.

The work was repeated in 1983, using protease to destroy the protein, by Naot and Remington, workers with some 15 years of publications in the field developing tests for toxoplasmosis parasite (Naot Y, Guptill D.R., Mullenax J. and Remington J.S. Infect. Immun. 41 No.1,331-338, 1983) but they were unable to obtain IgM or IgG specific reaction. In a further in-depth investigation of toxoplasmosis antigen Sharma et al J. of Immunology 131, No. 2, 977-983, 1983 could find no antigens, specific for IgM. We also attempted to repeat the work of Mineo's group but failed again using the ELISA tests to obtain specific IgM reaction with the protease digested antigen of Toxoplasma gondii.

EP-A-0189389 describes the use of an agglutination assay technique to determine IgM antibodies in a mixture of IgM and IgG antibodies. The IgM antibodies are differentiated from IgG antibodies by the addition of anti-IgG antibodies.

Conventional ELISA assays are effective only for measuring total immunoglobulin concentration, and cannot be satisfactorily used to measure either class of antibody individually in the presence of the other. This invention seeks to overcome that difficulty, and results from the observation that enzyme treatment of the antigen can enhance its reactivity with one class of antibody relative to the other.

The invention provides a method of assaying IgM antibodies to toxoplasmosis in a sample containing a mixture of immunoglobulins by the use of a toxoplasma antigen that has been modified by reaction with a proteolytic enzyme to enhance its reactivity with the IgM antibody relative to the other antibody, which method comprises incubating a mixture of the sample with the enzyme modified antigen and measuring by an agglutination technique the extent of binding of the IgM antibody to the enzyme modified antigen.

The invention is particularly suitable for assaying IgM antibodies in the presence of IgG antibodies.

The invention involves use of an antigen that has been reacted with a proteolytic enzyme in order to enhance its reactivity with the antibody being assayed relative to the other antibody. Suitable enzymes include proteases, such as for example, pronase and proteinase K. Reaction conditions are not very critical. For example, the antigen may be incubated with the enzyme, in a medium and at a pH at which both are stable, at 37°C for periods of a few minutes to a few hours.

Enzyme modification of the antigen enhances its reactivity with the antibody being assayed relative to the other antibody. This may be achieved by enhancing the reactivity of the antigen with the antibody being assayed in an absolute sense; or by decreasing the reactivity of the antigen with the other antibody; or by both effects. The reactivity of an antibody with its antigen is dependent upon the number of binding sites on each and the binding affinity of each site for the other component. Changing the reactivity of the antigen with an antibody may involve changing the number of binding sites, or changing the binding affinity of one or more of the sites, or both such effects.

The invention involves incubating a mixture of the sample with the enzyme modified antigen. Assay conditions can be conventional, as can the means for measuring the extent of binding of the antibody being assayed to the enzyme modified antigen. However, the enzyme modified antigen is preferably used in a form bound to a solid surface, which surface may be the wall of an assay vessel. Preferably, the enzyme modified antigen is bound to small solid particles which can be maintained in suspension for the duration of the incubation and, if required, subsequently brought down by centrifuging or magnetic means. The nature of the particulate material is not critical, there may be used glass spheres, gold sols or, particularly, latex (polystyrene) particles. Such particles are available on a commercial basis, and techniques for binding reagents such as antigens to them are well known in the art.

The extent of binding of the enzyme modified antigen to the antibody being assayed is preferably measured by an agglutination technique. The extent of agglutination may be determined by turbidimetry, but is preferably determined by a particle counting technique such as our IMPACT (trade mark) diagnostic system. This system involves counting particle numbers in a defined volume of liquid. The particles are of known uniform size, and the instrument is calibrated to count only particles approximately that size, so that assemblies of two or more particles formed by agglutination are rejected and not counted. It has been surprisingly found that, by the use of such a system, the selectivity of the enzyme modified antigen for the antibody being assayed, in relation to the other antibody, is greatly increased, so that the other antibody causes substantially no interference.

It is presently believed that the explanation for this surprising phenomenon is as follows. In an agglutination test of this kind, spheres, say latex spheres of 0.8 microns diameter, are held together by two to three antibodies whose diameters are of the order of 0.01 microns. With thermal motion and by flowing through the measuring system, shear forces are exerted on the latex aggregates tending to break them apart. Only aggregates where the immune binding force between antigen and antibody holding them together is sufficiently strong, can withstand these shear forces.

According to our observations with an ELISA method (see Example 1 below) treatment of Toxoplasma gondii antigen with proteinase K does not make the antigen completely specific for IgM. IgG continues to react after treatment, though to a lesser extent, and so the treatment of the antigen with proteinase K does not by itself give a completely specific assay for IgM antibodies.

However, the binding of IgG to the enzyme modified antigen is very much more reduced than that of IgM. The binding of IgG is reduced to about 1/10 of its original value, whereas that of IgM is reduced to 1/4 - 1/5. In contrast, in agglutination assays the binding of IgG to enzyme modified antigen is almost completely inhibited. We believe that the binding affinity of IgG (but not of IgM) for the enzyme modified antigen is so low that agglutinated particles are broken apart by the shear forces discussed above, and are thus counted as unagglutinated in a particle counting system.

The following Examples illustrate the invention.

## Example 1

### Preparation of toxoplasmosis antigen (Toxoantigen)

The toxoplasmosis antigen was prepared from the peritoneal exudate of mice infected two days previously with trophozoites of T.gondii.

The peritoneal cavity was washed with 3-5 ml of sterile physiological saline. To 1 ml samples of this suspension, 0.015 ml of the antibiotic Pentrexyl and 10 units of heparin were added and allowed to stand for 30 minutes at room temperature in tubes with conical bottoms. The tubes were then centrifuged for 4 minutes at 600 rpm, and the residue was discarded. The suspension was again centrifuged for 30 minutes at 4,000 rpm at 4°C and the supernatant was discarded. After adding to the pellet 2 ml of 1 M NH4 Cl to haemolyse the red cells, and centrifuged again at 4,000 rpm at 4°C the pellet was washed three times with saline and centrifuged again for 30 minutes at 4,000 rpm at 4°C.

The pellet was resuspended in 2 ml of the distilled water and then repeatedly frozen with dry ice-acetone and thawed, each time the solution being subjected to ultrasonic mixing. After centrifuging for 10 minutes at 3,000 rpm, the supernatant was removed and stored at -20°C.

### Assay Procedure

Serum samples which contained both IgG and IgM antibodies were tested in microtitre plates the surface of which were treated with 100 ul of Toxoplasma gondii antigen prepared as described.

The plates were incubated overnight at 4°C with the antigen and then washed five times with physiological saline containing 0.05% Tween® 20 ("Tween" is a registered trade mark.) To one half the plates was added 100 ul/well of TRIS HCl buffer (0.01M pH 7.5, 0.02M CaC1$_2$) containing proteinase K at a concentration of 1 mg/ml buffer and the plates incubated for two hours at 37°C. The remaining plates were treated with TRIS buffer only and incubated for the same time. After incubation all plates were washed five times with physiological saline/0.05% Tween® 20. Patient sera were serially diluted for IgM assay to concentrations of 1/500, 1/1000, 1/2000, 1/4000 and 1/8000 while those for IgG assay were diluted 1/50, 1/100, 1/200, 1/400, 1/800. To each well of the microtitre plate was added 50 ul of the diluted serum and the plate was allowed to incubate for 1 hour at room temperature and washed 5 times with physiological saline/0.05% Tween® 20. Into each well, as appropriate, 100 ul of peroxidase-labelled anti IgM (2mg/ml) or peroxidase labelled anti IgG (2.5 mg/ml) were added at pH 5 in the presence of hydrogen peroxide. After 20 minutes incubation at room temperature 25 ul of a 0.1 N sulphuric acid solution was added and the optical density was read. Table 1 shows the results. The higher the optical density, the greater the concentraton of antibody present in the serum. Similar results were obtained by treating the antigen with proteinase K prior to adsorption on microtitre plate wells.

<u>Table 1</u>

Optical density of 4 sera from patients infected with Toxoplasma gondii

A = in wells with whole antigen of Toxoplasma gondii

B = in wells with antigen of Toxoplasma gondii digested with proteinase K

| Sample | | IgM antibodies Titre 1/- | | | | | | IgG antibodies Titre 1/- | | | | | |
| | | Blank | 500 | 1000 | 2000 | 4000 | 8000 | Blank | 50 | 100 | 200 | 400 | 800 |
|---|---|---|---|---|---|---|---|---|---|---|---|---|---|
| 1 | A | 0.034 | 1.603 | 1.455 | 1.122 | 0.714 | 0.498 | 0.050 | 1.595 | 1.524 | 1.642 | 1.497 | 1.420 |
| | B | 0.024 | 0.421 | 0.294 | 0.201 | 0.129 | 0.085 | 0.030 | 0.191 | 0.140 | 0.086 | 0.064 | 0.056 |
| 2 | A | 0.042 | 1.289 | 1.383 | 1.007 | 0.794 | 0.508 | 0.067 | 1.577 | 1.656 | 1.630 | 0.224 | 1.111 |
| | B | 0.028 | 0.456 | 0.304 | 0.186 | 0.140 | 0.113 | 0.035 | 0.168 | 0.134 | 0.096 | 0.062 | 0.045 |
| 3 | A | 0.033 | 1.541 | 1.382 | 1.122 | 0.823 | 0.548 | 0.066 | 1.563 | 1.643 | 1.656 | 1.248 | 0.129 |
| | B | 0.021 | 0.484 | 0.307 | 0.241 | 0.173 | 0.120 | 0.030 | 0.268 | 0.197 | 0.127 | 0.087 | 0.064 |
| 4 | A | 0.045 | 1.493 | 1.382 | 1.035 | 0.795 | 0.512 | 0.069 | 1.520 | 1.576 | 1.595 | 1.273 | 1.412 |
| | B | 0.029 | 0.656 | 0.367 | 0.262 | 0.160 | 0.108 | 0.031 | 0.230 | 0.215 | 0.145 | 0.110 | 0.097 |

As can be seen, digestion of the antigen with proteinase K substantially reduces its reactivity, both with IgM and with IgG. However, the reduction is much greater with IgG than with IgM. In other words, the enzyme modification has enhanced the reactivity of the antigen with IgM relative to IgG.

Example 2

Preparation of latex-toxoantigen (Lxtoxo)

To 250 ul of carboxylated latex (10% w/v) (Lx), 2 ml of barbital buffered saline (BBS) (0.02M, pH 8.1) was added and centrifuged for 10 minutes at 10,000 rpm. Supernatant was removed, and 250 ul of BBS was added and cooled to 4°C. 250 ul of BBS containing 25 mg of carbodiimide (CDI) to yield a mixture of 10 mg CDI/100 ul Lx was then added, the solution being freshly prepared and kept at 4°C and incubated for 1 hour with vortexing at 4°C.

After being centrifuged at 4°C for 10 minutes at 10,000 rpm, it was subjected to ultrasonic vibration and added to the solution of toxoantigen (5 mg) in BBS at 4°C (volume approximately 0.5 ml

The suspension was then incubated at 4°C for 2-4 hours under vortex or for 16 hours on a roller.

The latex suspension was washed three times with Tris (0.1N HCl, pH 7.5 and 20 mM in CaCl$_2$) and ultrasonically mixed after each centrifugation. This is the preparation of Lx for the treatment with enzymes at pH 7.5. For pepsin treatment the Lx is suspended in HCl 0.15 M. The lxtoxo was incubated with various protease enzymes under the conditions set out in Table 2.

Table 2

| Treatment of Lx Ag Toxo with specific enzymes | | | | |
|---|---|---|---|---|
| Enzyme | Enzyme Conc. | pH | Incubation | Temp. |
| Lipase | 1 mg/ml | 7.5 | 2 hours | 37°C |
| Pepsin | 5 mg/ml | 1.5 | 10' | 37°C |
| Trypsin | 1 mg/ml | 7.5 | 2 hours | 37°C |
| Pronase | 1 mg/ml | 7.5 | 2 hours | 37°C |
| Proteinase K | 1 mg/ml | 7.5 | 2 hours | 37°C |

Heat aggregated IgG human IgG (10 mg/ml) was heated to 63°C for 30 minutes and diluted 1:1 GBS.

Assay Procedure

30 ul of a 1/50 dilution of patient serum in GBS/1% BSA (GBS = glycine buffered saline) were added to 30 ul of a solution containing 60 ug/ml of an IgG mouse monoclonal antibody directed against human IgM and 5 mg/ml of heat aggregated human IgG to absorb Clq and RF. To this solution was then added 30 ul of latex Lx toxo at a concentration of 0.05% w/v.

The mixture was incubated for 30 minutes under vortex mixing. The reaction was stopped by the addition of 2 ml of GBS and unagglutinated particles only were counted in a blood cell counter modified to count only unagglutinated particles.

The mouse monoclonal anti-human IgM antibody was included in the reaction mixture because it was found, unexpectedly, to enhance IgM antibody agglutination by a factor of more than four-fold. This helps to minimise IgG interference. The heat aggregated IgG is present in solution to eliminate residual interferences arising from samples which contain high levels of IgG antibodies and rheumatoid factor (RF).

Results

As shown in Table 3 the pronase and proteinase K treatments of the antigen-coated latex completely destroys the agglutination due to IgG antibodies and reinforces the agglutination of the IgM antibodies; a possible explanation for this reinforcement is that the proteolysis releases more "IgM antigens" from the protein shell surrounding the particles and renders them available to react with IgM antibodies.

## Table 3

| | Latex Control | Latex lipase | Latex pepsin | Latex trypsin | Latex pronase | Latex proteinase K |
|---|---|---|---|---|---|---|
| Anti-Toxoplasma IgG serum | Agglutin | Decreased Agglutin | Decreased Agglutin | Increased Agglutin | No Agglutin | No Agglutin |
| Anti-Toxoplasma IgM serum | Agglutin | Decreased Agglutin | Decreased Agglutin | Increased Agglutin | Strongly increased Agglutin | Strongly increased Agglutin |

### Example 3

In order to confirm these results, 40 IgM positive sera were tested with the proteinase K latex-toxoantigen described in Example 2: all sera agglutinated to latex. 21 high titre IgG sera were similarly tested, and none was found to agglutinate the latex.

### Example 4

139 patient sera were assayed for IgM by the method of Example 2 using the proteinase K treated latex toxoantigen. The results are set out in Table 4 and the results obtained using two commercially available assays are included for comparison. Table 5 gives further quantitative results obtained according to the method. The following terms are used in the Tables.

Prior Art A. This is a microtitre plate method. The plastic wells contain an antibody specific for human IgM antibodies. IgM antibodies are captured from patient samples. After washing the wells a suspension of washed trophozoites are added. Following incubation, the wells are examined by eye for the presence of immobilised trophozoites.

Prior Art B. This is an enzyme-labelled immunosorbent assay, based on Prior Art A. In place of intact trophozoite, solubilised antigen and an enzyme-labelled second antibody, specific for the antigen are used as the detection method.

Neg., Pos. and Doubt. are abbreviations for negative, positive and doubtful and represent the qualitative results of the assay.

No immune sera are from patients not infected with toxoplasmosis which should have low IgM and low IgG.

Immune sera should have high IgG and low IgM.

Infected sera are from patients where IgM concentration may be rising or falling. A follow-up test may be required to determine whether the infection is very recent or is moving towards an immune IgG stage.

Recent infection refers to sera from patients at a stage where IgM concentration has not yet passed its peak.

Congenital sera comes from patients with congenital toxoplasmosis.

As emphasized in Table 5, the method of the invention provides a very discriminating and sensitive test. The large number of positive results obtained on patients previously thought to have no immunity suggests that toxoplasmosis may exist in a substantial part of the population.

Table 4

| Patient Class | No.of Sera | INVENTION | | Prior Art A | | | Prior Art B | |
|---|---|---|---|---|---|---|---|---|
| | | Neg. | Pos. | Neg. | Doubt | Pos | Neg. | Pos. |
| Not immune | 35 | 24 | 11 | 35 | 0 | 0 | 35 | 0 |
| Immune | 72 | 25 | 47 | 32 | 7 | 33 | 67 | 5 |
| Infected | 17 | 1 | 16 | 1 | 2 | 14 | 11 | 6 |
| Recent Infection | 13 | 0 | 13 | 0 | 0 | 13 | 2 | 11 |
| Congenital | 2 | 0 | 2 | 0 | 0 | 2 | 0 | 2 |

Table 5

| Patient Class | No.of Sera | Negative | Positive | Mean | Range |
|---|---|---|---|---|---|
| | | | | (Arbitrary Units) | |
| Not immune | 35 | 24 | 11 | 2 | 0-12 |
| Immune | 72 | 25 | 47 | 18 | 0-115 |
| Infected | 17 | 1 | 16 | 67 | 0-500 |
| Recent Infection | 13 | 0 | 13 | 540 | 150-1000 |

## Claims

1. A method of assaying IgM antibodies to toxoplasmosis in a sample containing a mixture of immunoglobulins by the use of a toxoplasma antigen that has been modified by reaction with a proteolytic enzyme to enhance its reactivity with the IgM antibody relative to the other antibody, which comprises incubating a mixture of the sample with the enzyme modified antigen and measuring by an agglutination technique the extent of binding of the IgM antibody to the enzyme modified antigen.

2. A method as claimed in claim 1, wherein the antibody being assayed is an IgM antibody in a sample containing a mixture of IgM and IgG immunoglobulin.

3. A method as claimed in claim 1 or claim 2, wherein the proteolytic enzyme is proteinase K.

4. A method as claimed in claim 1 or claim 2, wherein the proteolytic enzyme is a pronase.

5. A method as claimed in any one of the preceding claims, wherein the antigen is Toxoplasma gondii.

6. A method as claimed in any one of the preceding claims, wherein the enzyme modified antigen is used in a form bound to a solid surface.

7. A method as claimed in claim 6, wherein the enzyme modified antigen is used in a form bound to latex particles.

8. A method as claimed in claim 7, wherein the antigen has been enzyme modified before being bound to the particles.

9. A method as claimed in claim 7, wherein the antigen has been enzyme modified after being bound to the particles.

10. A method as claimed in any of the preceding claims, wherein the agglutination technique involves counting unagglutinated particles.

**11.** A method as claimed in any of the preceding claims, wherein the agglutination is measured by turbidimetry.

**12.** A method as claimed in any of the preceding claims, wherein the agglutination is measured by nephelometry.

**13.** A method as claimed in any of the preceding claims, wherein the agglutination is measured by eye.

**Patentansprüche**

**1.** Verfahren zur Bestimmung von IgM-Antikörpern gegen Toxoplasmose in einer eine Mischung aus Immunglobulinen enthaltenden Probe durch Verwendung eines Toxoplasma-Antigens, das zur Erhöhung seiner Reaktionsfähigkeit mit dem IgM-Antikörper im Vergleich zum anderen Antikörper durch Riaktion mit einem protolytischen Enzym modifiziert worden ist, dadurch gekennzeichnet, daß man eine Mischung aus Probe und dem enzymmodifizierten Antigen inkubiert und die Bindung des IgM-Antikörpers an das enzymmodifizierte Antigen mittels eines Agglutinationsverfahrens bestimmt.

**2.** Verfahren nach Anspruch 1, dadurch gekennzeichnet, daß es sich bei dem zu bestimmenden Antikörper um einen IgM-Antikörper in einer Probe, die eine Mischung aus den Immunglobulinen Igm und IgG enthält, handelt.

**3.** Verfahren nach Anspruch 1 oder 2, dadurch gekennzeichnet, daß es sich bei dem proteolytischen Enzym um Proteinase K handelt.

**4.** Verfahren nach Anspruch 1 oder 2, dadurch gekennzeichnet, daß es sich bei dem proteolytischen Enzym um eine Pronase handelt.

**5.** Verfahren nach einem der vorhergehenden Ansprüche, dadurch gekennzeichnet, daß es sich bei dem Antigen um Toxoplasmagondii handelt.

**6.** Verfahren nach einem der vorhergehenden Ansprüche, dadurch gekennzeichnet, daß das verwendete enzymmodifizierte Antigen an eine feste Oberfläche gebunden ist.

**7.** Verfahren nach Anspruch 6, dadurch gekennzeichnet, daß das verwendete enzymmodifizierte Antigen an Latexteilchen gebunden ist.

**8.** Verfahren nach Anspruch 7, dadurch gekennzeichnet, daß die Enzymmodifizierung des Antigens vor dessen Bindung an die Teilchen vorgenommen wird.

**9.** Verfahren nach Anspruch 7, dadurch gekennzeichnet, daß die Enzymmodifizierung des Antigens nach Bindung an die Teilchen vorgenommen wird.

**10.** Verfahren nach einem der vorhergehenden Ansprüche, dadurch gekennzeichnet, daß beim Agglutinationsverfahren eine Zählung der nichtagglutinierten Teilchen vorgenommen wird.

**11.** Verfahren nach einem der vorhergehenden Ansprüche, dadurch gekennzeichnet, daß die Agglutination turbidimetrisch bestimmt wird.

**12.** Verfahren nach einem der vorhergehenden Ansprüche, dadurch gekennzeichnet, daß die Agglutination nephelometrisch bestimmt wird.

**13.** Verfahren nach einem der vorhergehenden Ansprüche, dadurch gekennzeichnet, daß die Agglutination visuell bestimmt wird.

**Revendications**

**1.** Procédé d'analyse d'anticorps IgM contre la toxoplasmose dans un échantillon contenant un mélange d'immunoglobulines au moyen d'un antigène de la toxoplasmose qui a été modifié par réaction avec une enzyme protéolytique pour augmenter sa réactivité avec l'anticorps IgM par rapport à d'autres anticorps, qui comprend l'incubation d'un mélange de l'échantillon avec l'antigène modifié par l'enzyme et la mesure par une technique d'agglutination de l'étendue du lien de l'anticorps IgM avec l'antigène modifié par l'enzyme.

2. Procédé suivant la revendication 1, où l'anticorps à analyser est un anticorps IgM dans un échantillon contenant un mélange d'immunoglobulines IgM et IgG.

3. Procédé suivant la revendication 1 ou la revendication 2, où l'enzyme protéolytique est la protéinase K.

4. Procédé suivant la revendication 1 ou la revendication 2, où l'enzyme protéolytique est une pronase.

5. Procédé suivant l'une quelconque des revendications précédentes, où l'antigène est le Toxoplasma gondii.

6. Procédé suivant l'une quelconque des revendications précédentes, où l'antigène modifié par l'enzyme est utilisé sous une forme liée à une surface solide.

7. Procédé suivant la revendication 6, où l'antigène modifié par l'enzyme est utilisé sous une forme liée à des particules de latex.

8. Procédé suivant la revendication 7, où l'antigène a été modifié par l'enzyme avant d'être fixé aux particules.

9. Procédé suivant la revendication 7, où l'antigène a été modifié par l'enzyme après être fixé aux particules.

10. Procédé suivant l'une quelconque des revendications précédentes, où la technique d'agglutination comprend le comptage des particules non agglutinées.

11. Procédé suivant l'une quelconque des revendications précédentes, où l'agglutination est mesurée par turbidimétrie.

12. Procédé suivant l'une quelconque des revendications précédentes, où l'agglutination est mesurée par néphélométrie.

13. Procédé suivant l'une quelconque des revendications précédentes, où l'agglutination est mesurée à l'oeil.